# EUROPÄISCHE PATENTSCHRIFT

(11) **EP 2 841 120 B1**
(45) Veröffentlichungstag und Bekanntmachung des Hinweises auf die Patenterteilung: **18.11.2020**
(21) Anmeldenummer: 13719277.9
(22) Anmeldetag: 23.04.2013
(51) Int. Cl.: A61M 1/00

(54) **BEFESTIGUNGSVORRICHTUNG FÜR EIN UNTERDRUCKTHERAPIEGERÄT**
FIXING DEVICE FOR A NEGATIVE PRESSURE THERAPY APPARATUS
DISPOSITIF DE FIXATION D'UN APPAREIL DE VACUOTHÉRAPIE

(30) Priorität: 26.04.2012 DE 102012008301
(43) Veröffentlichungstag der Anmeldung: 04.03.2015
(73) Patentinhaber: Paul Hartmann AG, 89522 Heidenheim (DE)
(72) Erfinder: CROIZAT, Pierre, 89542 Herbrechtingen (DE); ECKSTEIN, Axel, 89522 Heidenheim (DE); HOFSTETTER, Jürgen, 89522 Heidenheim (DE); MAHLER, Andreas, 89143 Blaubeuren-Asch (DE); WEINBERG, Andreas, 70176 Stuttgart (DE); RUF, Martin, 72070 Tübingen (DE); FONFARA-DÖRR, Astrid, 89077 Ulm (DE)
(74) Vertreter: Eibl, Christian
(86) Internationale Anmeldenummer: PCT/EP2013/001208
(87) Internationale Veröffentlichungsnummer: WO 2013/159904

(56) Entgegenhaltungen:
- EP-A2- 1 523 081
- WO-A1-2010/130022
- WO-A1-2011/045468
- WO-A2-2011/094306
- US-A- 4 874 118
- US-A- 5 244 175
- US-A- 5 850 954
- US-A1- 2005 242 144
- US-A1- 2007 032 763
- US-A1- 2009 145 938
- US-A1- 2009 182 275
- US-A1- 2009 308 823
- US-A1- 2011 168 857
- US-A1- 2011 247 994
- US-A1- 2011 284 601

## Beschreibung

Die Erfindung betrifft eine Befestigungsvorrichtung für ein Unterdrucktherapiegerät umfassend ein Haltemittel, einen Hohlkörper und einen Aufnahmebereich. Insbesondere betrifft die Erfindung eine derartige Befestigungsvorrichtung für ein durch den Benutzer tragbares und mitführbares Unterdrucktherapiegerät. Die Erfindung betrifft weiterhin ein Unterdrucktherapiegerät, welche eine Befestigungsvorrichtung umfasst.

Bei einem Unterdrucktherapiegerät handelt es sich um eine Vorrichtung zur Bereitstellung von Unterdruck für medizinische Anwendungen, insbesondere zur Unterdruckbehandlung von Wunden am menschlichen oder tierischen Körper.

Vorrichtungen zur Unterdruckbehandlung von Wunden am menschlichen oder tierischen Körper sowie für eine Unterdruckbehandlung von Wunden geeignete Unterdrucktherapiegeräte sind bereits mehrfach bekannt geworden, beispielsweise durch WO 1996/05873, US 2004/0073151, WO 2011/018132 oder WO-2011/018133.

Bei einer üblichen Vorrichtungen zur Unterdruckbehandlung von Wunden am menschlichen oder tierischen Körper kommuniziert ein Unterdrucktherapiegerät über eine Saugleitung mit der Wunde oder der Wundumgebung, wobei ein luftundurchlässiges Abdeckmaterial zum luftdichten Verschließen der Wunde und der Wundumgebung vorgesehen ist, so dass ein Unterdruck im Wundraum herstellbar und Flüssigkeiten aus dem Wundraum in einen Exsudatbehälter absaugbar sind. Das Unterdrucktherapiegerät umfasst üblicherweise einen Exsudatbehälter. Der Exsudatbehälter ist häufig an das Unterdrucktherapiegerät abnehmbar gekoppelt. Der Exsudatbehälter kann gleichfalls im Inneren des Unterdrucktherapiegerätes herausnehmbar untergebracht sein oder als separate Komponente vorliegen. Weiterhin sind insbesondere zum einmaligen Gebrauch vorgesehene Unterdrucktherapiegeräte bekannt, welche einen mit dem Unterdrucktherapiegerät unlösbar verbundenen Exsudatbehälter umfassen.

Aus dem Stand der Technik bekannte Unterdrucktherapiegeräte sind entweder stationäre Geräte, wie beispielsweise in der WO 1996/05873 beschrieben, oder am Körper des Benutzers tragbare und mitführbare Geräte für den mobilen Einsatz, wie beispielsweise in der WO 2009/047524 beschrieben. Des Weiteren sind Unterdrucktherapiegeräte bekannt, die sowohl zur Verwendung im stationären als auch im mobilen Einsatz vorgesehen sind, wie beispielsweise in der WO 2011/018132 und in der WO 2011/018133 beschrieben. So offenbaren die WO 2011/018132 und die WO 2011/018133 jeweils ein am Körper eines Benutzers tragbares Unterdrucktherapiegerät, wobei das Unterdrucktherapiegerät aus zwei Gehäuseteilen besteht. Die einen Unterdruck erzeugende Einrichtung ist in einem ersten Gehäuseteil des Unterdrucktherapiegerätes angeordnet. Ein nach Gebrauch wegwerfbarer Einweg-Exsudatbehälter (Kanister) zur Aufnahme von Körperflüssigkeiten bildet einen zweiten Gehäuseteil des Unterdrucktherapiegerätes. Die Gehäuseteile sind lösbar gegeneinander fixierbar. An den ersten Gehäuseteil des Unterdrucktherapiegerätes können hinsichtlich ihrer Größe und ihrer Aufnahmekapazitäten für Körperflüssigkeiten unterschiedlich ausgebildete zweite Gehäuseteile gekoppelt werden, wobei kleinere Kanister, beispielsweise mit einer Aufnahmekapazität von 300 ml, für den mobilen Einsatz vorgesehen sind und größere Kanister, beispielsweise mit einer Aufnahmekapazität von 800 ml, für den stationären Einsatz vorgesehen sind. Ein derartiges Unterdrucktherapiegerät ist unter der Bezeichnung VivanoTec ® (Paul Hartmann AG, Deutschland) kommerziell erhältlich.

Unterdrucktherapiegeräte zur Unterdruckbehandlung von Wunden kommen beispielsweise in Krankenhäusern, in Arztpraxen oder in der privaten Umgebung des Patienten zum Einsatz. Im Krankenhaus werden die Unterdrucktherapiegeräte unter anderem im Operationssaal oder im Bettenbereich der Klinik verwendet. Stationäre Unterdrucktherapiegeräte oder mobile Unterdrucktherapiegeräte im stationären Einsatz müssen dabei üblicherweise in unmittelbarer Nähe des Patienten untergebracht werden. Tragbare Unterdrucktherapiegeräte im mobilen Einsatz werden normalerweise am Körper des Patienten getragen oder beispielsweise in einer Tragetasche mitgeführt. Insbesondere beim stationären Einsatz ergibt sich das Problem einer sicheren Unterbringung des Unterdrucktherapiegerätes und der mit dem Gerät verbundenen Komponenten, beispielsweise den Stromversorgungskomponenten oder den Datenkommunikationskomponenten, sofern vorhanden. Das Unterdrucktherapiegerät oder die Stromversorgungs- bzw. Datenkommunikationskomponenten sollen das medizinische Personal bei der Behandlung des Patienten nicht behindern. Stromversorgungskomponenten umfassen häufig ein Netzkabel mit Netzstecker zur Verbindung mit dem Stromnetz, eine Stromversorgungskabel mit Gerätestecker zur Stromversorgung des Unterdrucktherapiegerätes und ein zwischen den beiden Kabeln angeordnetes Netzgerät (Transformator), welches die Netzspannung in die von dem Unterdrucktherapiegerät benötigte Spannung umwandelt. Als störend im medizinischen Behandlungsbereich erweisen sich häufig die zwischen Stromversorgungskabel und dem Netzkabel angeordneten Netzgeräte, welche typischerweise ohne weitere Befestigung am Boden des Behandlungsraums herumliegen. Ein lose auf dem Boden des Behandlungsraums liegendes Netzgerät kann im Krankenhausbereich, insbesondere im Operationssaal, eine Gefahrenquelle darstellen und sich hinsichtlich der Hygieneerfordernisse als problematisch erweisen.

In der Praxis hat es sich zudem gezeigt, dass gelegentlich übersehen wird, das Unterdrucktherapiegerät von seiner Stromversorgung zu trennen, wenn es transportiert werden soll. Hierbei können Unfälle entstehen, wenn durch eine mit dem Unterdrucktherapiegerät verbundene Stromversorgungskomponente oder Datenkommunikationskomponente andere im Behandlungsbereich vorhandene Gegenstände, beispielsweise Infusionsständer, umgerissen werden.

Häufig werden Unterdrucktherapiegeräte auf Tische gestellt, an Infusionsständer geklemmt, oder direkt am Krankenbett, beispielsweise an vertikal oder horizontal angeordneten Stangen, fixiert.

Im Stand der Technik sind Befestigungsvorrichtungen zur Fixierung von Unterdrucktherapiegeräten beschrieben. Beispielsweise offenbart die WO 2008/036344 eine Hängevorrichtung zur Befestigung eines medizinischen Gerätes, insbesondere zur Befestigung eines Unterdrucktherapiegerätes. Die Hängevorrichtung der WO 2008/036344 ist unmittelbar mit der Rückseite des Unterdrucktherapiegerätes verbunden und umfasst eine Klemmvorrichtung zur lösbaren Befestigung des Gerätes an vertikal oder horizontal angeordneten Stangen. Die Klemmvorrichtung wird durch einen Drehknopf betätigt. Zur Befestigung oder Entfernung des Unterdrucktherapiegerätes von der Stange muss das Unterdrucktherapiegerät mit einer Hand gehalten werden, während die andere Hand den Drehknopf festzieht oder löst. Die Hängevorrichtung eignet sich nicht zur Anbringung des Unterdrucktherapiegerätes an einer Platte. Eine sichere Unterbringung eines gegebenenfalls vorhandenen Netzgerätes ist im Zusammenhang mit der Hängevorrichtung nicht vorgesehen.

Die bisher unveröffentlichte deutsche Patentanmeldung DE102011011831.4 mit dem Titel "Vorrichtung zur Bereitstellung von Unterdruck für medizinische Anwendungen" beschreibt eine Halteeinrichtung zum lösbaren Befestigen eines Unterdrucktherapiegerätes an einer horizontal oder vertikal oder schräg geneigten Strebe oder Stange. Die Halteeinrichtung umfasst ein an dem Gehäuse des Unterdrucktherapiegerätes befestigbares starres Halteteil und ein vorzugsweise elastisch ausgebildetes bandförmiges Haltemittel, welches um eine Stange herumlegbar und auf sich oder auf das Halteteil zurückführbar und schließbar ist.

Die bisher unveröffentlichte deutsche Patentanmeldung DE102011076868.8 mit dem Titel "Vorrichtung zur Bereitstellung von Unterdruck zur Unterdruckbehandlung von Wunden mit einer Halte- oder Trageeinrichtung" beschreibt einen Halte- oder Tragegurt für ein tragbares Unterdrucktherapiegerät. Gemäß einer Ausführungsform der in der DE102011076868.8 offenbarten Erfindung umfasst der Halte- oder Tragegurt eine Verbindungseinrichtung zur Verbindung des Halte- oder Tragegurts mit einem externen Träger, beispielsweise mit einem Infusionsständer oder mit einer Bettstrebe.

Auch bei den in der DE102011011831.4 oder in der DE102011076868.8 beschriebenen Befestigungsvorrichtungen muss das Unterdrucktherapiegerät beim Befestigen oder Entfernen von einer Stange mit einer Hand gehalten werden, während die andere Hand den Befestigungsmechanismus schließt und öffnet. Die Vorrichtungen eigenen sich gleichfalls nicht zur Anbringung des Unterdrucktherapiegerätes an einer Platte. Eine sichere Unterbringung eines gegebenenfalls vorhandenen Netzgerätes ist im Zusammenhang mit den in der DE102011011831.4 oder in der DE102011076868.8 beschriebenen Befestigungsvorrichtungen nicht vorgesehen.

Der vorliegenden Erfindung liegt die Aufgabe zugrunde, eine Befestigungsvorrichtung zur lösbaren Befestigung eines Unterdrucktherapiegerätes dahingehend zu verbessern, dass sie in noch benutzerfreundlicherer Weise handhabbar und bedienbar ist.

Insbesondere liegt der vorliegenden Erfindung die Aufgabe zu Grunde, eine einfach zu bedienende, flexible und sichere Befestigungsvorrichtung für ein zumindest optional tragbares Unterdrucktherapiegerät zur Verfügung zu stellen. Die Befestigungsvorrichtung soll die Befestigung des Unterdrucktherapiegerätes an im Behandlungsbereich eines Patienten vorhandenen geeigneten Gegenständen ermöglichen, beispielsweise an einer Stange, an einer Strebe, an einer Platte oder an einem vorspringenden Bereich. Wünschenswert wäre es zudem, wenn am Unterdrucktherapiegerät vorhandene Stromversorgungskomponenten oder Datenkommunikationskomponenten sicher untergebracht sind und nicht im Behandlungsbereich herumliegen.

Diese Aufgabe wird erfindungsgemäß gelöst durch eine Vorrichtung zur Unterdrucktherapie von Wunden nach Anspruch 1, umfassend ein Unterdrucktherapiegerät sowie eine Befestigungsvorrichtung für ein Unterdrucktherapiegerät.

, Die Vorrichtung umfasst ein Haltemittel zum Fixieren der Vorrichtung an einem Gegenstand, einen Hohlkörper in den eine oder mehrere Stromversorgungskomponenten und/oder Datenkommunikationskomponenten verbringbar sind sowie einen Aufnahmebereich zum Aufnehmen des Unterdrucktherapiegerätes. Bei dem Gegenstand handelt es sich insbesondere um eine Stange, um eine Strebe, um eine Platte oder um einen vorspringenden Bereich.

Der Hohlkörper umfasst eine oder mehrere Öffnungen zum Durchführen von Stromkabeln oder Kommunikationskabeln.

Die erfindungsgemäße Vorrichtung ermöglicht eine sichere Unterbringung und Befestigung eines Unterdrucktherapiegerätes an Gegenständen, welche sich in unmittelbarer Umgebung des Patienten befinden, beispielsweise an einer Bettstrebe, an einem Infusionsständer, an einer Tischplatte oder an einem Bettgalgen. Die erfindungsgemäße Vorrichtung eignet sich gleichermaßen zur sicheren Aufbewahrung des Unterdrucktherapiegerätes, beispielsweise in einem Vorratsraum des Krankenhauses. Die erfindungsgemäße Vorrichtung kann dabei in vorteilhafter Weise im Sinne einer Ladestation gleichzeitig zum Aufbewahren des Unterdrucktherapiegerätes und zum Aufladen von in tragbaren Unterdrucktherapiegeräten vorhandenen Akkumulatoren eingesetzt werden. Vorteilhaft können Stromversorgungskomponenten, insbesondere das Netzgerät und Abschnitte des Stromversorgungskabels, sicher und ordentlich im Hohlkörper der Befestigungsvorrichtung untergebracht werden. Weiterhin hat es sich als vorteilhaft erwiesen, dass die von dem Unterdrucktherapiegerät benötigten Stromversorgungskomponenten sicher an der Befestigungseinrichtung fixierbar sind. Somit entfällt eine mögliche Gefahrenquelle, welche dann entstehen kann, wenn es ein Benutzer beim Wegtragen des Unterdrucktherapiegerätes versäumt, das Gerät vorher von der Stromversorgungskomponente zu trennen. Wie eingangs dargestellt können solchenfalls Unfälle entstehen, wenn durch eine mit dem Unterdrucktherapiegerät verbundene Stromversorgungskomponente oder Datenkommunikationskomponente andere im Behandlungsbereich vorhandene Gegenstände umgerissen werden. Ein derartiger Unfall ist bei Verwendung der erfindungsgemäßen Befestigungsvorrichtung vermeidbar, da die Stromversorgungskomponenten und/oder die Datenkommunikationskomponenten an der Befestigungsvorrichtung fixierbar sind und solchenfalls nicht lose im Behandlungsbereich herumliegen. Versäumt es ein Benutzer beim Transport des Unterdrucktherapiegerätes die Stromversorgungskomponente oder eine Datenkommunikationskomponenten zu entfernen, so löst sich bei Verwendung der erfindungsgemäßen Befestigungsvorrichtung üblicherweise die Steckverbindung zwischen Stromversorgungs- bzw. Datenkabel und Unterdrucktherapiegerät, ohne Schaden an der Halterung oder an dem Unterdrucktherapiegerät zu verursachen.

Unter Aufnahmebereich wird im Zusammenhang mit der Erfindung derjenige Bereich der Befestigungsvorrichtung verstanden, welcher das Unterdrucktherapiegerät aufnimmt, also lösbar festhält.

Gemäß einer wesentlichen Ausführungsform der Erfindung umfasst das Haltemittel Bereiche, welche eine formschlüssige, eine kraftschlüssige oder eine stoffschlüssige Fixierung der Vorrichtung an einem Gegenstand ermöglichen. Entsprechend kann die Fixierung der erfindungsgemäßen Befestigungsvorrichtung an einem Gegenstand insbesondere durch Klemmen, Einrasten, Einhaken, Verschrauben, Vernageln, Verklammern, Vernieten, Verschweißen, Verschmelzen, Verkleben oder durch magnetische Anziehungskraft erfolgen.

Vorzugsweise erfolgt die Fixierung der Befestigungsvorrichtung an einem Gegenstand durch Klemmen, Einrasten oder Einhaken, da eine derartige Befestigung einfach und schnell zu bewerkstelligen ist.

Für viele Anwendungen erweist es sich als besonders vorteilhaft, wenn das Haltemittel einen Klemmmechanismus umfasst, so dass die Befestigungsvorrichtung kraftschlüssig an einem Gegenstand fixiert werden kann, insbesondere an einer Stange, an einer Platte oder an einem vorspringenden Bereich. Gemäß dieser besonders bevorzugten Ausführungsform umfasst das Haltemittel insbesondere eine Halteschelle, eine Bügelschelle, eine Bandschelle, eine Federklemme oder eine Zwinge, insbesondere eine Schraubzwinge. Die Befestigungsvorrichtung kann somit auf einfache Weise und schnell an einem Gegenstand befestigt oder von dem Gegenstand entfernt werden.

Damit das Haltemittel insbesondere an einer Stange oder an einer Strebe formschlüssig und kraftschlüssig angeklemmt werden kann, umfasst der Klemmmechanismus vorzugsweise einen Wellenklemmblock oder Backen mit längsförmig ausgebildeten Mulden. Ein solchermaßen ausgestaltete Klemmmechanismus kann dabei gleichermaßen zum Anklemmen der Befestigungsvorrichtung an einer Platte oder am vorspringenden Bereichen verwendet werden, da die im Wellenklemmblock oder in den Backen vorhandenen längsförmigen Mulden hierbei nicht störend sind.

Am Klemmmittel gegebenenfalls vorhandene Backen umfassen vorzugsweise an ihrer Oberfläche ganz oder teilweise eine Gummiauflage, so dass die kraftschlüssige Fixierung des Klemmmittel an einer Stange, an einer Strebe oder an einer Platte verbessert wird, wobei gleichzeitig eine Beschädigung der Stange oder Platte vermieden wird.

Gemäß einer weiteren wesentlichen Variante der Erfindung erfolgt die Fixierung der Befestigungsvorrichtung an einem Gegenstand durch Verschrauben, Vernageln, Verklammern, Vernieten, Verschweißen, Verschmelzen oder Verkleben. Eine derartige Fixierung der Befestigungsvorrichtung ist dann vorteilhaft, wenn die Befestigungsvorrichtung zum dauerhaften Verbleiben an einem bestimmten Ort vorgesehen ist. So kann es beispielsweise wünschenswert sein, eine erfindungsgemäße Befestigungsvorrichtung an solchen Bereichen im Krankenhaus dauerhaft anzubringen, in denen eine Unterdrucktherapie von Wunden häufig durchgeführt wird. Dabei kann es sich beispielsweise um stationäre Bereiche der Klinik handeln, welche auf die Behandlung schwerer chronischer Wunden spezialisiert sind. Die Befestigungsvorrichtung könnte auch an einer im Operationssaal vorhandenen Vorrichtung befestigt werden, beispielsweise durch Verschraubungen, welche zur Bereithaltung weiterer während der Operation benötigter medizinischer Geräte vorgesehen ist. Es erweist sich dabei als vorteilhaft, dass die erfindungsgemäße Vorrichtung die für den Betrieb des Unterdrucktherapiegerätes benötigten Stromversorgungskomponenten bereit halten kann, wobei einzelne Stromversorgungskomponenten, beispielsweise das Netzgerät und/oder Abschnitte des Stromversorgungskables sicher im Hohlkörper der Befestigungsvorrichtung aufbewahrt werden. Ein weiterer Vorteil einer permanent im Operationssaal vorhandenen erfindungsgemäßen Befestigungsvorrichtung besteht darin, dass eine ansonsten beim Einbringen der Vorrichtung in den Operationsbereich notwendige Sterilisation der Vorrichtung unterbleiben kann. Anstelle einer Fixierung durch Verschrauben wäre es gleichermaßen möglich, die Vorrichtung auf eine andere Art mit einem Gegenstand zu verbinden, beispielsweise durch Verkleben oder Verschweißen. Eine derartige unlösbare Fixierung des Haltemittels an einem Gegenstand ist im Rahmen der vorliegenden Erfindung im Sinne einer Ausführungsform umfasst.

Erfindungsgemäß umfasst die Befestigungsvorrichtung einen Hohlkörper in den eine oder mehrere Stromversorgungskomponenten verbringbar sind, wobei es sich bei der Stromversorgungskomponente insbesondere um ein Netzgerät und/oder um einen Kabelabschnitt und/oder um eine elektrische Steckverbindung handelt. Alternativ oder zusätzlich ist es möglich, in den Hohlraum eine oder mehrere Datenkommunikationskomponenten einzubringen. Bei den Datenkommunikationskomponenten handelt es sich insbesondere um ein Datenkommunikationskabel oder um Stecker. Es ist jedoch gleichfalls möglich, dass in den Hohlraum aktive oder passive funktionelle Komponenten zur Datenkommunikation eingebracht werden, beispielsweise eine Hub, ein Relaismodul oder eine Komponente zur Anbindung an ein WLAN-Netz.

Erfindungsgemäß ist es erforderlich, dass die Befestigungsvorrichtung weiterhin einen Einschub zur Aufnahme einer oder mehrerer Stromversorgungs- oder Datenkommunikationskomponenten umfasst, wobei der Einschub in den Innenraum des Hohlkörpers einbringbar ist. Der Einschub sollte hinsichtlich seiner Form auf die aufzunehmen Stromversorgungs- oder Datenkommunikationskomponenten abgestimmt sein. Falls die Stromversorgungskomponente beispielsweise ein Netzgerät umfasst, welches in den Hohlkörper der Befestigungsvorrichtung eingebracht werden soll, so sollte der Einschub das Netzgerät aufnehmen können. Das Netzgerät kann somit zunächst vom Benutzer der Befestigungsvorrichtung in den Einschub eingesteckt werden. Danach wird der Einschub mit dem Netzgerät in den Hohlraum der Befestigungsvorrichtung eingebracht. Hierbei erweist es sich als besonders vorteilhaft, wenn der Einschub eine Kabelaufwicklung umfasst, so dass nicht benötigte Kabelabschnitte von Stromversorgungs- oder Datenkommunikationskabeln untergebracht werden können.

Der Boden des Aufnahmebereichs kann zumindest teilweise durch den in den Hohlraum eingebrachten Einschub gebildet werden. Dies kann beispielsweise dann der Fall sein, wenn der Einschub über eine im Aufnahmebereich angeordnete Öffnung in den Hohlkörper einbringbar ist. Der Einschub kann dann durch den Aufnahmebereich hindurch in den Hohlkörper eingebracht werden. Der Einschub kann dabei so ausgebildet sein, dass er nach dem Einbringen in den Hohlkörper gleichsam einer Schublade den Hohlkörper verschließt.

Insgesamt erweist es sich als vorteilhaft, wenn die Öffnung zum Einbringen oder Herausnehmen von Stromversorgungs- oder Datenkommunikationskomponenten oder die Öffnung zum Einbringen oder Herausnehmen des Einschubes derart angeordnet ist, dass die Öffnung nach der Aufnahme des Unterdrucktherapiegerätes durch das Gerät bedeckt und somit verschlossen ist.

Nach einem weiteren Erfindungsgedanken von wesentlicher Bedeutung erweist es sich als vorteilhaft, dass der Aufnahmebereich der Befestigungsvorrichtung eine zum Unterdrucktherapiegerät komplementäre Form aufweist, so dass das aufzunehmende Unterdrucktherapiegerät weitestgehend unverrückbar im Aufnahmebereich gehalten wird. Der Aufnahmebereich kann hierzu beispielsweise die Form einer Schale aufweisen, in welcher der untere Bereich des Unterdrucktherapiegerätes formschlüssig gehalten werden kann.

Grundsätzlich können bei der Befestigungsvorrichtung Aufnahmebereich und Hohlkörper insgesamt einstückig ausgebildet sein oder alternativ jeweils von einem oder mehreren separaten Bauteilen gebildet werden.

Gemäß einer bevorzugten Ausführungsform der Erfindung werden Aufnahmebereich und Hohlkörper insgesamt durch ein einziges Bauteil gebildet. Gemäß dieser bevorzugten Ausführungsform sind der Hohlkörper und der Aufnahmebereich entsprechend einstückig ausgebildet, insbesondere sind der Hohlkörper und der Aufnahmebereich einstückig aus demselben Material ausgebildet. Das den Hohlkörper und den Aufnahmebereich umfassende Bauteil kann beispielsweise aus Kunststoff durch Spritzguss in einem Schritt hergestellt werden. Solchenfalls kann ein oberer Teil des Hohlkörpers gleichzeitig zumindest einen Teilbereich des Bodens des Aufnahmebereichs bilden.

Nach einer weiteren besonders bevorzugten Ausführungsform der Erfindung werden Hohlkörper und Aufnahmebereich aus zwei oder mehreren separaten Bauteilen gebildet, wobei die Bauteile zur Herstellung der Befestigungsvorrichtung dann vorzugsweise durch Verschrauben, Verkleben oder Verschweißen miteinander verbunden werden. Die Bauteile können hierbei jeweils aus demselben Material oder jeweils aus einem oder mehreren unterschiedlichen Materialien hergestellt werden.

Nach einem weiteren Erfindungsgedanken wird vorgeschlagen, dass die Befestigungsvorrichtung ein mit dem Unterdrucktherapiegerät lösbar oder unlösbar verbundenes Kopplungselement umfasst. Der Aufnahmebereich ist bei dieser Ausführungsform derart ausgebildet, dass zumindest das Kopplungselement im Aufnahmebereich aufgenommen werden kann. Es ist auch möglich, dass Kopplungselement und ein mit dem Kopplungselement verbundener Teil des Unterdrucktherapiegerätes im Aufnahmebereich aufgenommen werden können.

Ein Kopplungselement zur Verwendung mit einer Befestigungsvorrichtung, wie in der vorliegenden Offenbarung beschrieben, ist daher gleichfalls Gegenstand der vorliegenden Erfindung.

Insbesondere ist es vorteilhaft, wenn der Aufnahmebereich eine zu dem Kopplungselement oder zu dem Kopplungselement und einem damit verbundenen Unterdrucktherapiegerät komplementäre Form aufweist.

Bei dem Kopplungselement kann es sich beispielsweise um ein plattenförmiges Element handeln, welches mit dem Boden des Unterdrucktherapiegerätes lösbar verbunden werden kann. Die äußere Form (Umriss) des Kopplungselementes kann dabei der äußeren Form (Umriss) des Bodens des Unterdrucktherapiegerätes weitgehend entsprechend. Zur lösbaren Verbindung des Kopplungselementes mit dem Unterdrucktherapiegerät ist am Unterdrucktherapiegerät vorzugsweise eine Aufnahme für das Kopplungselement vorgesehen. Bei der Aufnahme kann es sich beispielsweise um ein im Boden des Unterdrucktherapiegerätes vorhandenes Gewinde handeln, so dass das Kopplungselement über eine am Kopplungselement vorhandene Schraube am Unterdrucktherapiegerät befestigt werden kann. Das Kopplungselement kann dauerhaft mit dem Unterdrucktherapiegerät verbunden bleiben. Gegebenenfalls am Boden des Unterdrucktherapiegerätes vorhandene Elemente, beispielsweise Gummifüßchen, können auch auf der Unterseite des Kopplungselementes vorhanden sein, damit das Unterdrucktherapiegerät, wenn es sich nicht in der Befestigungsvorrichtung befindet, mit an dem Gerät befestigtem Kopplungselement auf einer ebenen Fläche abgestellt werden kann.

Gemäß einer vorteilhaften Ausführungsform der Erfindung ist der Aufnahmebereich weitgehend plattenförmig ausgebildet, wobei die Ebene der Platte bei Gebrauch der Vorrichtung horizontal angeordnet ist.

Gemäß einer weiteren, besonders bevorzugten Ausführungsform umfasst die Befestigungsvorrichtung ein mit dem Unterdrucktherapiegerät unlösbar verbundenes Kopplungselement. Nach einer Weiterführung dieses speziellen Erfindungsgedankens stellt das Kopplungselement einen Gehäuseteil des Unterdrucktherapiegerätes dar. Das Unterdrucktherapiegerät ist also bei dieser Ausführungsform hinsichtlich seiner äußeren Form auf den Aufnahmebereich der Befestigungsvorrichtung abgestimmt.

Nach einem unabhängigen Erfindungsgedanken erweist es sich als vorteilhaft, wenn der Aufnahmebereich ein Verschlussmittel umfasst, so dass das Unterdrucktherapiegerät lösbar arretiert werden kann.

Bei dem Verschlussmittel kann es sich beispielsweise um einen Riemen, um einen Sperrhebel, um einen Sperrstift oder um einen einhackenden Mechanismus handeln. Denkbar wäre auch ein Magnetverschluss.

Das Verschlussmittel sorgt für eine sichere Verbindung von Befestigungseinrichtung mit dem Unterdrucktherapiegerät, so dass das Unterdrucktherapiegerät nicht herunterfallen kann.

Gemäß einer einfachen Ausführungsform kann es sich bei dem Verschlussmittel um einen an der Befestigungsvorrichtung vorhandenen Riemen handeln, welcher zur Sicherung des Unterdrucktherapiegerätes um das Gehäuse des Gerätes gelegt wird.

Vorzugsweise handelt es sich bei dem Verschlussmittel um ein einrastendes Verschlussmittel, welches das Unterdrucktherapiegerät lösbar arretieren kann.

Das Verschlussmittel kann dabei am Gehäuse des Unterdrucktherapiegerätes oder - sofern vorhanden - am Kopplungselement ansetzen.

Gemäß einer besonders bevorzugten Ausführungsform weist das Kopplungselement und/oder das Unterdrucktherapiegerät eine oder mehrere Ausnehmungen auf, in welche das am Aufnahmebereich vorhandene Verschlussmittel eingreifen kann. Insbesondere weist das Kopplungselement eine Ausnehmung auf, in welche das am Aufnahmebereich vorhandene Verschlussmittel eingreifen kann.

Das Verschlussmittel ist in weiterer Ausbildung der Erfindung durch ein manuell bedienbares Betätigungsorgan in Freigaberichtung bringbar. Vorzugsweise ist das manuell bedienbare Betätigungsorgan am Aufnahmebereich randständig und an der dem Haltemittel gegenüberliegenden Seite der Befestigungsvorrichtung vorgesehen. Das Betätigungsorgan ist insbesondere als eindrückbarer Taster ausgebildet oder ein herausziehbarer, beispielsweise ringförmig ausgebildeter Griff, in den ein einzelner Finger eingreifen kann.

Idealerweise sollte es möglich sein, das Unterdrucktherapiegerät mit nur einer Hand in der Befestigungsvorrichtung zu befestigen oder aus der Befestigungsvorrichtung zu lösen (Einhandbedienbarkeit).Gemäß einer besonders bevorzugten Ausführungsform kann das Unterdrucktherapiegerät deshalb mit einer Hand an der Befestigungsvorrichtung fixiert und/oder von der Befestigungsvorrichtung gelöst werden.

Gemäß einer weiteren vorteilhaften Weiterbildung sind im Aufnahmebereich ein oder mehrere Anschlagelemente vorhanden, so dass das Kopplungselement beim Inkontaktbringen mit dem Aufnahmebereich formschlüssig ausgerichtet wird. Vorzugsweise umfasst das eine oder die mehreren Anschlagelemente eine oder mehrere Ausnehmungen, in welche eine oder mehrere am Kopplungselement vorhandene Federn oder vorstehende Bereiche eingreifen können, so dass das Kopplungselement beim Inkontaktbringen mit dem Aufnahmebereich formschlüssig ausgerichtet wird.

Im Hinblick auf ein benutzerfreundliches Fügen von Unterdrucktherapiegerät und Befestigungsvorrichtung erweist es sich als vorteilhaft, wenn das Unterdrucktherapiegerät von oben leicht schräg zur Vertikalen an ein oder mehrere Anschlagelemente angesetzt wird dann nach unten drückbar ist, bis ein einrastendes Verschlussmittel - sofern vorhanden - einrastet, wobei das Unterdrucktherapiegerät in die Vertikale gebracht wird. Das Einrasten des Verschlussmittels ist normalerweise mit einem deutlich hörbaren Klick-Geräusch verbunden, so der Benutzer eine akustische Rückmeldung erhält, wenn das Unterdrucktherapiegerät in der Befestigungsvorrichtung arretiert ist.

Um das Einsetzen und Ausrichten des Unterdrucktherapiegerätes in der Befestigungsvorrichtung zu erleichtern, weist der Aufnahmebereich randständig gemäß einer weiteren bevorzugten Ausführungsform einen teilweise oder vollständig den Aufnahmebereich umlaufenden Steg oder Wulst auf, so dass das Kopplungselement beim Inkontaktbringen mit dem Aufnahmebereich formschlüssig ausgerichtet wird.

Mehrere Anschlagelemente und ein den Aufnahmebereich teilweise oder vollständig umlaufender Steg oder Wulst können gemäß einer weiteren, besonders bevorzugten Ausführungsform gleichzeitig im Aufnahmebereich vorhanden sein. Um die Einhandbedienbarkeit beim Herausnehmen des Unterdrucktherapiegerätes aus der Befestigungsvorrichtung zu verbessern, wäre es in diesem Zusammenhang denkbar, den Aufnahmebereich des Weiteren solchermaßen zu gestalten, dass das Unterdrucktherapiegerät aus dem Aufnahmebereich heraus gedrückt wird, sobald und solange das einrastende Verschlussmittel gelöst ist. Dies kann beispielsweise dadurch erreicht werden, dass im Aufnahmebereich ein Element vorhanden ist, welches eine Rückstellkraft bewirkt, beispielsweise eine Feder. Das Unterdrucktherapiegerät wird dann nach dem Lösen des einrastenden Verschlussmittels geringfügig aus dem Aufnahmebereich gedrückt, verbleibt jedoch aufgrund der im Aufnahmebereich vorhanden Anschlagelemente und/oder des den Aufnahmebereich umlaufenden Randes auf der Befestigungseinrichtung, ohne herabfallen zu können. Beim Einsetzen des Unterdrucktherapiegerätes in die Befestigungsvorrichtung muss das Gerät bei dieser besonders bevorzugten Ausführungsform gegen den Widerstand des eine Rückstellkraft bewirkenden Elementes in den Aufnahmebereich hineingedrückt werden, bis das Verschlussmittel einrastet. Das Einrasten des Verschlussmittels ist normalerweise mit einem deutlich hörbaren Klick-Geräusch verbunden.

Gemäß der vorstehend beschriebenen, in der praktischen Anwendung besonders vorteilhaften Ausführungsform, umfasst der Aufnahmebereich ein oder mehrere Anschlagelemente und/oder einen den Aufnahmebereich umlaufenden Rand, ein einrastendes Verschlussmittel sowie ein eine Rückstellkraft bewirkendes und das Unterdrucktherapiegerät aus dem Aufnahmebereich heraus drückendes Element, so dass das Unterdrucktherapiegerät beim Inkontaktbringen mit dem Aufnahmebereich gegen eine Rückstellkraft gedrückt werden muss, bis das Verschlussmittel einrastet.

Um die Befestigungsvorrichtung an jeder Art von im Krankenbereich vorhandenen Stangen, Streben, Platten oder vorspringenden Bereichen fixieren zu können, kann der Aufnahmebereich gegenüber dem Haltemittel verdreht werden. Die Befestigungsvorrichtung umfasst ein Drehgelenk, so dass der Aufnahmebereich gegenüber dem Haltemittel verdreht werden kann. Vorzugsweise ist das Drehgelenk einrastbar ausgestaltet. Gemäß einer besonders bevorzugten Ausführungsform kann das Drehgelenk in 0°-, 45°-, 90°-, 135°-, 180°, 225°-, 270°- oder 315°- Stellung lösbar einrasten, so dass die Vorrichtung an einer vertikal stehenden, an einer horizontal stehenden oder an einer schräg angeordneten Stange oder Platte angebracht werden kann.

Das auf das Drehgelenk wirkende und eine Einrastung des Drehgelenks bewirkenden Mittel (Drehsperre) umfasst vorzugsweise einen Hebel, einen Bügel oder einen Eindrückknopf. Insbesondere ist der Hebel, der Bügel oder der Eindrückknopf solchermaßen gestaltet, dass er mit einer Hand gelöst werden kann.

Gemäß einer besonders bevorzugten Ausführungsform kann das Drehgelenk in 0°-, 90°-, 180°- oder 270°-Stellung lösbar einrasten, so dass die Vorrichtung an einer vertikal stehenden oder an einer horizontal stehenden Stange, Strebe oder Platte angebracht werden kann, insbesondere an einer Tischplatte, an einer vertikalen Bettstange, an einem horizontalem Element eines Bettrahmens oder an einem Infusionsständer.

Mit dem Aufnahmebereich ist somit gleichfalls der mit dem Aufnahmebereich fest verbundene Hohlkörper gegenüber dem Haltemittel drehbar. Das Drehgelenk kann durch eine Verblendung abgedeckt werden, insbesondere durch eine Verblendung, welche das Drehgelenk gehäuseartig umschließt.

Gemäß einer Weiterführung des Erfindungsgedankens wäre es möglich in den Aufnahmebereich einen Stecker für die Stromversorgung derart zu integrieren, dass das Unterdrucktherapiegerät beim Einsetzen in die Befestigungsvorrichtung mit der Stromversorgung verbunden wird. Erforderlich ist hierzu ein geeignetes Gegenstück am Unterdrucktherapiegerät. Alternativ oder zusätzlich wäre es gleichermaßen möglich, eine Schnittstelle für Datenaustausch, insbesondere eine USB-Schnittstelle, in den Aufnahmebereich der Befestigungsvorrichtung derart zu integrieren, dass das Unterdrucktherapiegerät beim Einsetzen in die Befestigungsvorrichtung mit der Datenkommunikationsleitung verbunden wird. Gemäß der hier beschriebenen speziellen Ausführungsform der Erfindung dient die Befestigungsvorrichtung somit nicht nur der Fixierung des Unterdrucktherapiegerätes, sondern auch der Anbindung des Unterdrucktherapiegerätes an eine Stromversorgung und/oder an einer Datenkommunikationsleitung im Sinne einer "Docking Station".

Die erfindungsgemäße Befestigungsvorrichtung kann aus gebräuchlichen, für den vorbeschriebenen Zweck geeigneten Werkstoffen gefertigt werden. Üblicherweise werden die Komponenten aus Kunststoff und/oder aus Metall gefertigt. Zur Herstellung des Aufnahmebereiches und des Hohlkörpers eignen sich besonders Kunststoffe. Das Haltemittels und das Drehgelenk können zumindest teilweise aus einem Metall gefertigt werden.

In der praktischen Anwendung kann der Benutzer zunächst die Befestigungsvorrichtung an einem geeigneten, im Bereich des Patienten vorhandenen Gegenstand befestigen, beispielsweise durch Anklemmen an einer Bettstrebe. Anschließend kann der Benutzer Stromversorgungskomponenten oder Datenkommunikationskomponenten in den Hohlkörper der Befestigungsvorrichtung einbringen, wobei das Netzkabel, das Gerätestromversorgungskabel und/oder gegebenenfalls vorhandene Datenkommunikationskabel aus dem Hohlkörper durch Öffnungen im Hohlkörper nach außen geführt werden. Insbesondere kann der Benutzer das Netzgerät im Einschub - befestigen und überschüssige Kabelabschnitte um die Kabelaufwicklung (sofern vorhanden) des Einschubes legen. Der Einschub wird sodann in den Hohlkörper eingebracht. Das Unterdrucktherapiegerät wird in Kontakt mit dem Aufnahmebereich der Befestigungsvorrichtung gebracht und gegebenenfalls befestigt, beispielsweise durch ein rastendes Verschlussmittel (sofern vorhanden). Das aus dem Inneren des Hohlkörpers nach außen geführte Gerätestromversorgungskabel wird mit dem Unterdrucktherapiegerät verbunden. Das Unterdrucktherapiegerät ist nun einsatzbereit und in unmittelbarer Nähe zum Patienten sicher untergebracht.

Die vorliegende Erfindung betrifft eine Vorrichtung zur Unterdrucktherapie von Wunden, umfassend ein Unterdrucktherapiegerät sowie eine Befestigungsvorrichtung wie in der vorliegenden Anmeldung beschrieben.

Die vorliegende Erfindung betrifft insbesondere eine Vorrichtung zur Unterdrucktherapie von Wunden, umfassend ein am Körper eines Benutzers tragbares Unterdrucktherapiegerät sowie eine Befestigungsvorrichtung wie in der vorliegenden Anmeldung beschrieben.

Die vorliegende Erfindung betrifft weiterhin eine Vorrichtung zur Unterdrucktherapie von Wunden, umfassend ein am Körper eines Benutzers tragbares Unterdrucktherapiegerät sowie eine Befestigungsvorrichtung wie in der vorliegenden Anmeldung beschrieben, wobei das Unterdrucktherapiegerät einen nach Gebrauch wegwerfbaren Einweg-Exsudatbehälter zur Aufnahme von Körperflüssigkeiten, insbesondere von aus einer Wunde abgesaugten Wundsekreten und einen Anschluss für eine hierfür zum Körper führende Saugleitung umfasst. Vorzugsweise ist die den Unterdruck erzeugende Einrichtung in oder an einem ersten Gehäuseteil des Unterdrucktherapiegerätes angeordnet, während der Exsudatbehälter den zweiten Gehäuseteil des Unterdrucktherapiegerätes bildet oder in oder an dem zweiten Gehäuseteil angeordnet ist. Die Gehäuseteile sind dabei lösbar gegeneinander fixierbar. Vorzugsweise weist das Unterdrucktherapiegerät Befestigungsmittel auf, so dass es am Körper des Benutzers tragbar und mitführbar ist.

Gemäß einer besonders bevorzugten Ausführungsform ist der erste Gehäuseteil im am Körper des Benutzers mitgeführten Zustand des Unterdrucktherapiegerät (mobiler Betrieb) körperabgewandt und der zweite Gehäuseteil körperzugewandt vorgesehen, wobei beide Gehäuseteile im Wesentlichen scheibenförmig ausgebildet sind und über eine im Wesentlichen vertikal orientierte Trennebene gegeneinander anliegen.

Gemäß einer weiteren besonders bevorzugten Ausführungsform sind die Gehäuseteile des Unterdrucktherapiegerätes dabei durch ein schnappendes, rastendes oder in sonstiger Weise formschlüssig wirkendes Verriegelungs- oder Hintergriffsmittel gegeneinander lösbar fixierbar. Weiterhin ist das Verriegelungs- oder Hintergriffsmittel gemäß dieser weiteren besonders bevorzugten Ausführungsform durch ein manuell bedienbares Betätigungsorgan in Freigaberichtung bringbar und der erste und/oder zweite Gehäuseteil für einen manuellen Eingriff ausgebildet. Das Unterdrucktherapiegerät oder der erste oder der zweite Gehäuseteil kann ergriffen und der eine Gehäuseteil von dem anderen Gehäuseteil abgenommen werden. Da das manuell bedienbare Betätigungsorgan für das Verriegelungs- oder Hintergriffsmittel im Bereich des manuellen Eingriffs vorgesehen ist, kann ein Benutzer mit nur einer Hand das Verriegelungs- oder Hintergriffsmittel freigeben und dabei gleichzeitig den einen Gehäuseteil ergreifen und vom anderen Gehäuseteil lösen und abnehmen.

Gemäß einer besonders bevorzugten Ausführungsform betrifft die vorliegende Erfindung eine Vorrichtung zur Unterdrucktherapie von Wunden, umfassend ein Unterdrucktherapiegerät, wie in den Patentanmeldungen WO2011/018132 oder WO2011/018133 beschrieben sowie eine Befestigungsvorrichtung wie in der vorliegenden Anmeldung beschrieben. Auf den Inhalt der Patentanmeldungen WO2011/018132 oder WO2011/018133 wird hiermit ausdrücklich Bezug genommen.

Die vorliegende Erfindung umfasst des Weiteren ein Verfahren zum Befestigen eines Unterdrucktherapiegerätes, umfassend
- Bereitstellen einer Befestigungsvorrichtung
- Bereitstellen eines Unterdrucktherapiegerätes
- Bereitstellen eines Einschubes
- Fixieren der Befestigungsvorrichtung an einem Gegenstand, beispielsweise an einer Platte oder an einer Stange
- Verdrehen des Aufnahmebereiches gegenüber dem Haltemittels, so dass der Aufnahmebereich horizontal ausgerichtet ist
- Einstecken des Netzgerätes in den Einschub und gegebenenfalls Legen nicht benötigter Kabelabschnitte um die Kabelaufwicklung des Einschubes
- Einbringen von Stromversorgungskomponenten und/oder Datenkommunikationskomponenten in den Hohlkörper, wobei das Netzkabel, das Gerätestromversorgungskabel und/oder gegebenenfalls vorhandene Datenkommunikationskabel aus dem Inneren des Hohlkörpers durch Öffnungen im Hohlkörper nach außen geführt werden
- In Kontakt bringen des Unterdrucktherapiegerätes mit dem Aufnahmebereich der Befestigungsvorrichtung
- Optional: Sichern des Unterdrucktherapiegerätes im Aufnahmebereich, vorzugsweise durch ein einrastendes Verschlussmittel
- Verbinden des Gerätestromversorgungskabels mit dem Unterdrucktherapiegerät

Eine zur Wundbehandlung von Patienten einsatzbereite Vorrichtung zur Unterdrucktherapie von Wunden umfasst mindestens ein Unterdrucktherapiegerät, einen Unterdruck-Wundverband, ein Unterdruckanschlussstück (Port) und einen den Wundverband mit dem Unterdrucktherapiegerät verbindenden Drainageschlauch.

Weitere Merkmale, Einzelheiten und Vorteile der Erfindung ergeben sich aus den beigefügten Patentansprüchen und aus der zeichnerischen Darstellung und nachfolgenden Beschreibung bevorzugter Ausführungsformen der erfindungsgemäßen Vorrichtung. Für die Merkmale der Patentansprüche wird jeweils separat und losgelöst von einer Rückbeziehung der Ansprüche in beliebiger Kombination Schutz in Anspruch genommen. In der Zeichnung zeigt:
Figuren 1 A und B verschiedene Ansichten einer ersten bevorzugten Ausführungsform der erfindungsgemäßen Befestigungsvorrichtung mit einem daran befestigten Unterdrucktherapiegerät.
Figuren 2 A bis C verschiedene Ansichten einer weiteren bevorzugten Ausführungsform der erfindungsgemäßen Befestigungsvorrichtung mit einem daran befestigten Unterdrucktherapiegerät.
Figur 3 A ein zum Einbringen in den Innenraum des Hohlkörpers vorgesehener Einschub.
Figur 3 B eine erfindungsgemäße Befestigungsvorrichtung wie in Figur 2 gezeigt, wobei der Einschub sich nicht im Hohlkörper befindet. Auf die Darstellung des an der Befestigungsvorrichtung vorhandenen Haltemittels wurde verzichtet.
Figur 3 C eine erfindungsgemäße Befestigungsvorrichtung wie in Figur 2 gezeigt, wobei sich der Einschub im Hohlkörper befindet. Auf die Darstellung des an der Befestigungsvorrichtung vorhandenen Haltemittels wurde verzichtet.
Figur 4 A eine erfindungsgemäße Befestigungsvorrichtung wie in Figur 2 gezeigt, in der Aufsicht, wobei sich der Einschub im Hohlkörper befindet. Auf die Darstellung des an der Befestigungsvorrichtung vorhandenen Haltemittels wurde verzichtet.
Figur 4 B und C verschiedene Ansichten des Kopplungselementes.
Figur 4 D eine erfindungsgemäße Befestigungsvorrichtung wie in Figur 2 gezeigt, in der Aufsicht, wobei das Kopplungselement im Aufnahmebereich vorhanden ist. Auf die Darstellung des an der Befestigungsvorrichtung vorhandenen Haltemittels wurde verzichtet.
Figur 4 E ein Unterdrucktherapiegerät mit einem an dem Gerät befestigten Kopplungselement.

### Figurenbeschreibung

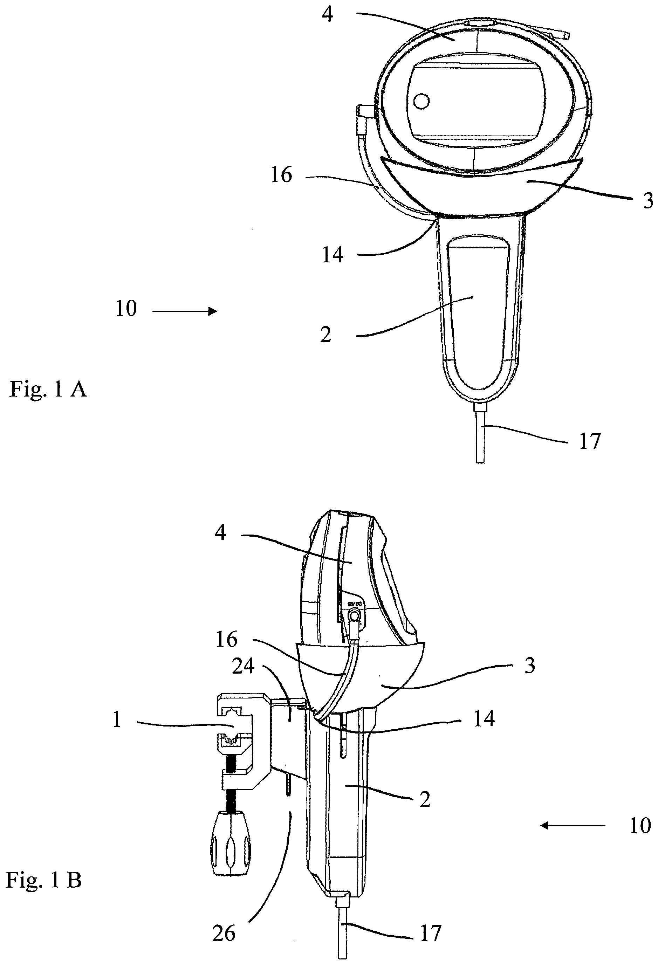

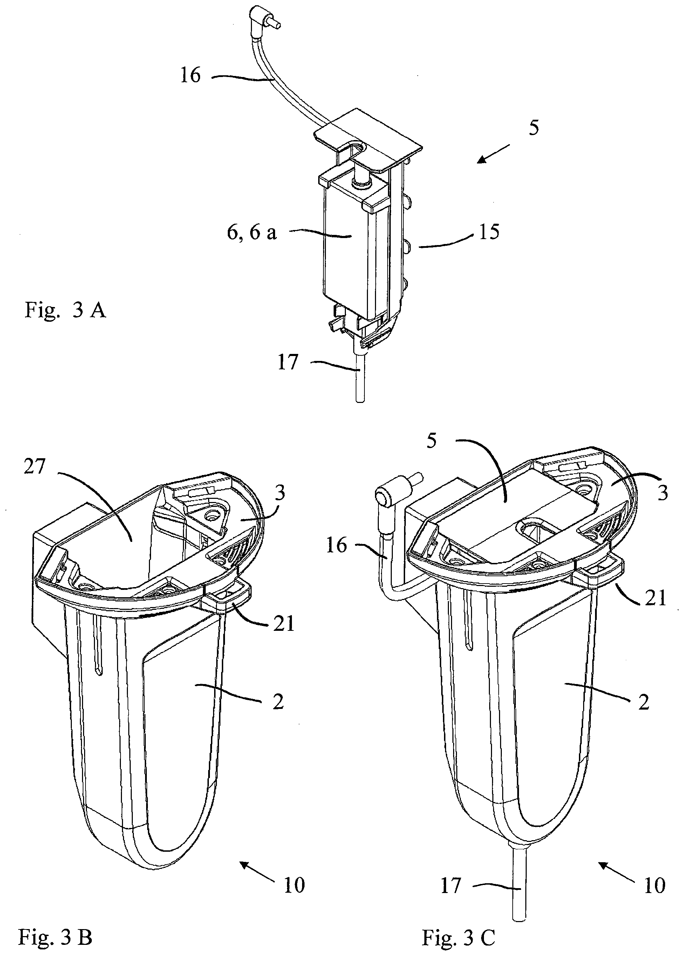

Figur 1 A zeigt eine erste bevorzugte Ausführungsform der erfindungsgemäßen Befestigungsvorrichtung 10 mit einem daran befestigtem Unterdrucktherapiegerät 4 von vorne. Das Unterdrucktherapiegerät 4 liegt weitestgehend unverrückbar in einem schalenartig ausgebildeten Aufnahmebereich 3, wobei der Aufnahmebereich 3 eine zum unteren Teil des Unterdrucktherapiegerätes 4 komplementäre Form aufweist. Nach unten hin schließt sich an den Aufnahmebereich 3 der Hohlkörper 2 an. In den Hohlkörper sind Stromversorgungskomponenten 6, beispielsweise ein Netzgerät 6 a (in Figur 1 A nicht sichtbar, siehe Figur 3 A) und nicht benötigte Kabelabschnitte, eingebracht. Das Netzkabel 17 wird durch eine Öffnung am unteren Ende des Hohlkörpers 2 aus dem Hohlkörper 2 nach außen geführt. Das Gerätestromversorgungskabel 16, welches das Netzgerät 6 a mit dem Unterdrucktherapiegerät 4 verbindet, wird durch die Öffnung 14 aus dem Hohlkörper 2 nach außen geführt. Das Haltemittel zum Fixieren der Befestigungsvorrichtung an einem Gegenstand ist in Figur 1 A nicht sichtbar, da das Haltemittel von dem Hohlkörper 2 und dem Aufnahmebereich 3 vollständig verdeckt ist.

Die in Figur 1A von vorne gezeigte erste bevorzugte Ausführungsform der Befestigungsvorrichtung ist in Figur 1 B in Seitenansicht dargestellt, so dass das Haltemittel 1 erkennbar ist. In der in den Figuren 1 A und B gezeigten Ausführungsform ist das Haltemittel 1 als Klemmmittel ausgebildet. Die Befestigungsvorrichtung 10 kann mittels des in Form einer Schraubzwinge ausgestalteten Haltemittels 1 an Stangen, Streben, Platten oder vorspringenden Bereichen lösbar montiert werden. Der Aufnahmebereich 3 und der mit dem Aufnahmebereich verbundene Hohlkörper 2 sind gegenüber dem Haltemittel 1 drehbar, da zwischen Hohlkörper 2 und dem Haltemittel 1 ein Drehgelenk angeordnet ist. Das Drehgelenk ist durch eine Verblendung 24 abgedeckt und deshalb in Figur 1 B nicht sichtbar. Die Verblendung 24 umschließt das Drehgelenk gehäuseartig.

Das Drehgelenk kann mittels einer Drehsperre vorzugsweise in 0°-, 45°-, 90°-, 135°-, 180°, 225°-, 270°, 315°- Stellung lösbar einrasten. Die Drehsperre kann mit einem Bedienelement entriegelt werden. Bei dem in Figur 1 B gezeigtem Ausführungsbeispiel handelt es sich bei dem Bedienelement um den nach unten hin abstehenden Hebel 26, welcher mit einer Hand gelöst werden kann. Somit ist die Befestigungsvorrichtung 10 an jeder Art von vertikal, horizontal oder schräg angeordneten Stange oder Platte befestigbar.

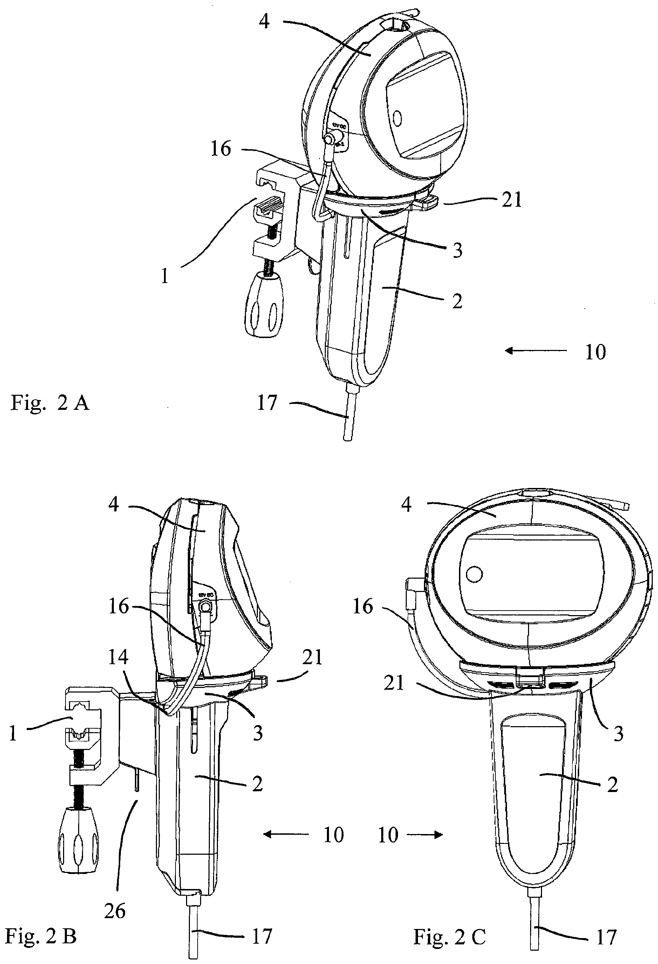

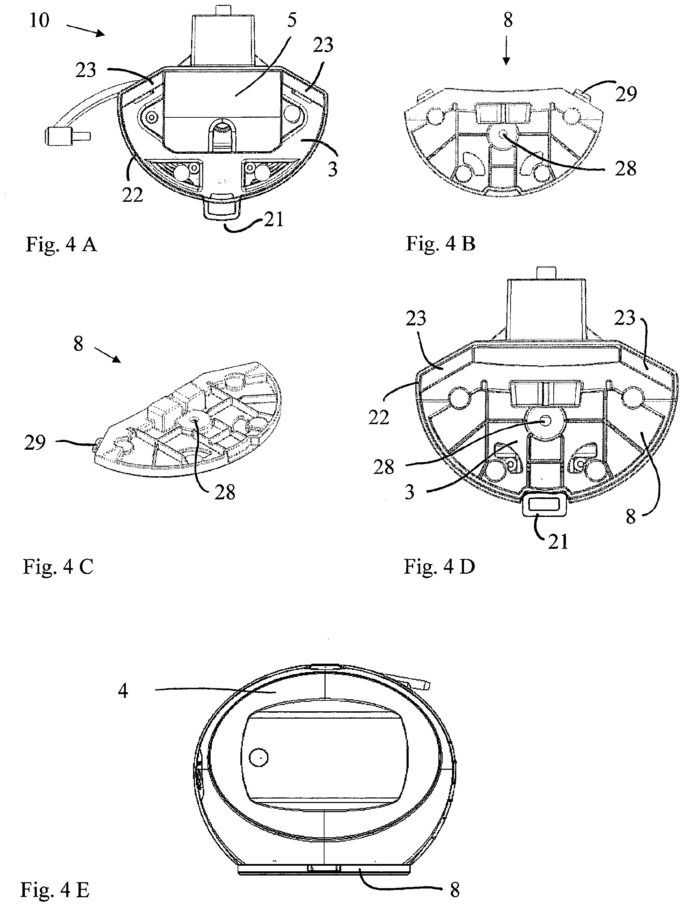

Die Figuren 2 A bis C zeigen eine weitere bevorzugte Ausführungsform der erfindungsgemäßen Befestigungsvorrichtung 10 mit einem daran befestigten Unterdrucktherapiegerät 4 von schräg vorne (Figur 2 A), von der Seite (Figur 2 B) sowie von vorne (Figur 2 C). Der Aufnahmebereich 3 ist bei dem in Figur 2 A - C gezeigtem Ausführungsbeispiel gegenüber dem in den Figuren 1 A und B gezeigtem Ausführungsbeispiel flacher ausgebildet. Der Aufnahmebereich 3 ist bei der bevorzugten Ausführungsform gemäß Figuren 2 A bis C zur Aufnahme eines Kopplungselementes (in Figuren 2 A bis C nicht sichtbar, siehe Figuren 4 B bis E, Bezugszeichen 8) vorgesehen. Des Weiteren umfasst der Aufnahmebereich 3 ein einrastendes Verschlussmittel mit einem Griff 21, so dass das Unterdrucktherapiegerät 4 und das mit dem Unterdrucktherapiegerät 4 verbundene Kopplungselement lösbar im Aufnahmebereich 3 der Befestigungsvorrichtung 10 arretiert werden kann. Wie bei dem in den Figuren 1 A und B gezeigtem Beispiel können im Inneren des Hohlkörpers 2 Stromversorgungskomponenten untergebracht werden. Hierzu befindet sich im Inneren des Hohlkörpers 2 ein Einschub (in den Figuren 2 A bis C nicht sichtbar) zur Aufnahme von Stromversorgungskomponenten. Die Figuren 2 A bis C lassen weiterhin das aus dem Hohlkörper 2 durch eine am unteren Ende des Hohlkörpers 2 vorhandene Öffnung herausgeführte Netzkabel 17 sowie das durch eine weitere Öffnung 14 im Hohlkörper 2 herausgeführte Gerätestromversorgungskabel 16 erkennen. In den Figuren 2 A und 2 B ist das in Figur 2 C durch den Hohlkörper 2 und den Aufnahmebereich 3 verdeckte Haltemittel 1 erkennbar. Entsprechend Figur 1 A und B ist das Haltemittel 1 in Form einer Schraubzwinge ausgebildet, so dass die Befestigungsvorrichtung 10 beispielsweise an einer Stange oder Bettstrebe angeschraubt werden könnte. Das Haltemittel 1 umfasst Backen, in denen länglich ausgebildete Mulden vorhanden sind. Derartige Mulden erleichtern das Anbringen des Haltemittels 1 an stangenartigen Gegenständen.

Zwischen dem Haltemittel 1 und dem Hohlkörper 2 ist wie bei dem in den Figuren 1 A und B gezeigtem Ausführungsbeispiel ein lösbar einrastendes Drehgelenk vorhanden, wobei das Drehgelenk gleichfalls durch eine Verblendung 24 abgedeckt ist.

Das Drehgelenk kann mittels einer Drehsperre vorzugsweise in 0°-, 45°-, 90°-, 135°-, 180°, 225°-, 270°, 315°- Stellung lösbar einrasten. Eine Entriegelung der Drehsperre erfolgt mittels des Hebels 26.

Die Öffnung 14 ist nur bei der seitlichen Darstellung in Figur 2 B erkennbar. Eine weitere Öffnung kann an der gegenüberliegenden Seite vorhanden sein.

Figur 3 A zeigt den zum Einbringen in den Innenraum des Hohlkörpers 2 vorgesehenen Einschub 5. Der Einschub 5 ist zur Aufnahme einer oder mehrerer Stromversorgungskomponenten 6 (beispielsweise ein Netzgerät 6 a, ein Kabel oder eine elektrische Steckverbindung) und/oder Datenkommunikationskomponenten (beispielsweise ein Datenkommunikationskabel, eine Komponente zur Anbindung an ein WLAN-Netz oder ein Stecker) vorgesehen. Bei der in Figur 3 A gezeigten Ausführungsform umfassen die mittels des Einschubes 5 in das Innere des Hohlkörpers 2 zu verbringenden Stromversorgungskomponenten 6 ein Netzgerät 6 a, welches am Einschub 5 befestigt ist sowie Kabelabschnitte des Netzkabels 17 und Kabelabschnitte des Gerätestromversorgungskabel 16. Zur Befestigung des Netzgerätes 6 a dienen am Einschub 5 vorhandene Halteelemente. Der Einschub 5 ist bei der hier vorgestellten Ausführungsform aus Kunststoff hergestellt.

Der Einschub 5 ist derart ausgebildet, dass er formschlüssig in den Innenraum des Hohlkörpers 2 eingeschoben werden kann. Bei der hier gezeigten Ausführungsform wird der Einschub 5 durch die Öffnung 27 (siehe Figur 3 B) in den Innenraum des Hohlkörpers 2 eingeführt. Vor dem Einführen des Einschubs 5 in den Hohlkörper muss das Netzkabel 17 durch eine am unteren Ende des Hohlkörpers 2 vorhandene Öffnung durch den Hohlkörper hindurchgeführt werden. Ebenso muss das Gerätestromversorgungskabel 16 durch die Öffnung 14 (siehe Figur 2 B) geführt werden.

In Figur 3 A ist eine auf der Rückseite des Einschubes 5 vorhandene Kabelaufwicklung 15 erkennbar. Die Kabelaufwicklung 15 dient der Unterbringung nicht benötigter Kabelabschnitte (beispielsweise Abschnitte des Gerätestromversorgungskabels).

In Figur 3 B ist die in den Figuren 2 A bis C gezeigte Ausführungsform der Befestigungsvorrichtung 10 ohne den darin befindlichen Einschub 5 gezeigt. Erkennbar ist die Öffnung 27 zur Aufnahme des Einschubes 5. Figur 3 C zeigt die Befestigungsvorrichtung 10 nach dem Einführen des Einschubes 5 in das Innere des Hohlkörpers 2. Die Öffnung 27 ist durch eine am oberen Abschnitt des Einschubes 5 vorhandene Platte vollständig verschlossen. Die Befestigungsvorrichtung 10 umfasst weiterhin ein Haltemittel. Bei den Figuren 3 B und 3 C wurde jedoch der Übersichtlichkeit halber auf die Darstellung des Haltemittels 1 (siehe Figur 2 A und B) verzichtet.

Figur 4 A zeigt die in den Figuren 2 A bis C gezeigte Ausführungsform der Befestigungsvorrichtung 10 von oben, wobei das Unterdrucktherapiegerät 4 in der zeichnerischen Darstellung entfernt wurde. Auf die Darstellung des gleichwohl vorhandenen Haltemittels 1 (siehe Figur 2 A und 2 B) wurde der Übersichtlichkeit halber verzichtet. Bei der in Figur 4 A gezeigten Befestigungsvorrichtung 10 wurde der Einschub 5 in das Innere des Hohlkörpers 2 eingeführt, so dass die Öffnung 27 (siehe Figur 3 B) durch den oberen Teil des Einschubes 5 vollständig verschlossen ist. Erkennbar sind ein um den Aufnahmebereich 3 laufender Steg 22 sowie die seitlich angeordneten Anschlagelemente 23. Der Steg 22 und die Anschlagelemente 23 erleichtern das Anbringen und das formschlüssige Ausrichten des an der Befestigungsvorrichtung 10 anzubringenden Unterdrucktherapiegerätes 4. Das einrastende Verschlussmittel umfasst einen ringartig ausgebildeten Griff 21, so dass das Verschlussmittel zum Entfernen des Unterdrucktherapiegerätes 4 durch Ziehen an dem ringartig ausgebildeten Griff 21 entriegelt werden kann.

Das Kopplungselement 8 ist in den Figuren 4 B und 4 C dargestellt. Das Kopplungselement 8 umfasst eine Schraube (in den Figuren 4 B und 4 C nicht dargestellt), welche zum Anbringen des Kopplungselementes 8 am Boden des Unterdrucktherapiegerätes 4 vorgesehen ist. Dazu wird die Schraube durch die Bohrung 28 geführt und an dem Unterdrucktherapiegerät festgeschraubt. Weiterhin umfasst das Kopplungselement 8 gemäß einer besonders vorteilhaften Ausführungsform eine oder mehrere Vorsprünge bzw. Federelemente 29. Diese greifen beim Aufsetzen des mit einem Kopplungselement 8 versehenen Unterdrucktherapiegerätes jeweils in eine im Aufnahmebereich vorzugsweise randständig vorhandene Öffnung bzw. Nut ein. Gemäß einer besonders bevorzugten Ausführungsform der Befestigungsvorrichtung 10 ist an jedem der beiden Anschlagelemente 23 jeweils eine Nut (in den Figuren 2 bis 4 nicht sichtbar) zur Aufnahme der am Kopplungselement vorhandenen Federn 29 vorgesehen.

Figur 4 D zeigt die Befestigungsvorrichtung 10 zur Veranschaulichung der Passung von Kopplungselement 8 und Aufnahmebereich 3 mit eingesetztem Kopplungselement 8 von oben (normalerweise befindet sich das Kopplungselement 8 jedoch am Unterdrucktherapiegerät 4). Erkennbar ist, dass das Kopplungselement 8 mittels der Anschlagelemente 23 und dem umlaufenden Rand 22 formschlüssig im Aufnahmebereich 3 gehalten wird.

Beim Einsetzen des mit dem Kopplungselement 8 verbundenen Unterdrucktherapiegerätes 4 in die Befestigungsvorrichtung 10 rastet das Verschlussmittel in das Kopplungselement 8 ein. Durch Ziehen an dem zum Verschlussmittel gehörenden Griff 21 (zur Hervorhebung wurde der ringförmig ausgebildete Griff 21 in Figur 4 D freiliegend dargestellt) wird das Verschlussmittel entriegelt bzw. entsperrt und gibt das Unterdrucktherapiegerät 4 frei, so dass es abgenommen werden kann.

Figur 4 E zeigt ein Unterdrucktherapiegerät 4 mit einem an dem Gerät befestigten Kopplungselement 8. Bei der hier gezeigten besonders vorteilhaften Ausführungsform der Erfindung wird das Kopplungselement 8 mit dem Unterdrucktherapiegerät 4 verschraubt. Hierzu befindet sich an der Unterseite des Unterdrucktherapiegerätes 4 ein Gewinde.

Das Kopplungselement 8 kann im normalen Betrieb des Unterdrucktherapiegerätes stets mit dem Unterdrucktherapiegerät verbunden bleiben. Aufgrund der ebenen Unterseite des Kopplungselementes 8 kann das Unterdrucktherapiegerät 4 jederzeit mit einem daran befestigten Kopplungselement 8 beispielsweise auf einer ebenen Fläche abgestellt oder am Körper eines Benutzers getragen werden. Bei dem Unterdrucktherapiegerät 4 handelt es sich insbesondere um ein am Körper eines Benutzers tragbares Unterdrucktherapiegerät, beispielsweise um die Unterdrucktherapieeinheit VivanoTec® des Herstellers Paul Hartmann AG.

## Patentansprüche

1. Vorrichtung zur Unterdrucktherapie von Wunden, umfassend ein Unterdrucktherapiegerät (4) sowie eine Befestigungsvorrichtung (10) für das Unterdrucktherapiegerät (4), wobei die Befestigungsvorrichtung
- ein Haltemittel (1) zum Fixieren der Befestigungsvorrichtung (10) an einem Gegenstand, und
- einen Aufnahmebereich (3) zum Aufnehmen des Unterdrucktherapiegerätes (4), umfasst, **dadurch gekennzeichnet,**
**dass** die Befestigungsvorrichtung einen Einschub (5) zur Aufnahme einer oder mehrerer Stromversorgungskomponenten (6) und/oder Datenkommunikationskomponenten umfasst,
und **dass** die Befestigungsvorrichtung (10) einen Hohlkörper (2) umfasst, in den eine oder mehrere Stromversorgungskomponenten (6) und/oder Datenkommunikationskomponenten verbringbar sind, wobei der Hohlkörper (2) eine oder mehrere Öffnungen (14) zum Durchführen von Kabelabschnitten umfasst, wobei der Einschub (5) in den Innenraum des Hohlkörpers (2) einbringbar ist und weiter dadurch,
**dass** die Befestigungsvorrichtung (10) ein Drehgelenk umfasst, so dass der Aufnahmebereich (3) gegenüber dem Haltemittel (1) verdreht werden kann.

2. Vorrichtung zur Unterdrucktherapie von Wunden nach Anspruch 1, wobei das Haltemittel (1) Bereiche umfasst, die eine formschlüssige, eine kraftschlüssige oder eine stoffschlüssige Fixierung der Befestigungsvorrichtung an einem Gegenstand ermöglichen, insbesondere durch Klemmen, Einrasten, Einhaken, Verschrauben, Vernageln, Verklammern, Vernieten, Verschweißen, Verschmelzen, Verkleben oder durch magnetische Anziehungskraft.

3. Vorrichtung zur Unterdrucktherapie von Wunden nach Anspruch 1 oder 2, wobei das Haltemittel (1) einen Klemmmechanismus umfasst, so dass die Befestigungsvorrichtung (10) kraftschlüssig an einem Gegenstand fixiert werden kann, insbesondere an einer Stange, an einer Strebe, an einer Platte oder an einem vorspringenden Bereich.

4. Vorrichtung zur Unterdrucktherapie von Wunden nach einem der vorhergehenden Ansprüche, wobei das Haltemittel (1) eine Halteschelle, eine Bügelschelle, eine Bandschelle, eine Federklemme oder eine Zwinge, insbesondere eine Schraubzwinge, umfasst.

5. Vorrichtung zur Unterdrucktherapie von Wunden nach einem der vorhergehenden Ansprüche, wobei es sich bei der Stromversorgungskomponente (6) um ein Netzgerät (6 a) und/oder um einen Abschnitt eines Kabels und/oder um eine elektrische Steckverbindung handelt.

6. Vorrichtung zur Unterdrucktherapie von Wunden nach einem der vorhergehenden Ansprüche, wobei der Einschub (5) eine Kabelaufwicklung (15) umfasst.

7. Vorrichtung zur Unterdrucktherapie von Wunden nach einem der vorhergehenden Ansprüche, wobei der Aufnahmebereich (3) eine zum Unterdrucktherapiegerät (4) komplementäre Form aufweist, so dass das Unterdrucktherapiegerät (4) weitestgehend unverrückbar im Aufnahmebereich (3) gehalten wird.

8. Vorrichtung zur Unterdrucktherapie von Wunden nach einem der vorhergehenden Ansprüche, weiterhin umfassend ein mit dem Unterdrucktherapiegerät (4) lösbar verbindbares oder unlösbar verbundenes Kopplungselement (8), wobei der Aufnahmebereich (3) das Kopplungselement (8) aufnehmen kann.

9. Vorrichtung zur Unterdrucktherapie von Wunden nach einem der vorhergehenden Ansprüche, wobei der Aufnahmebereich (3) ein Verschlussmittel umfasst, so dass das Unterdrucktherapiegerät (4) lösbar an der Befestigungsvorrichtung (10) arretiert werden kann, wobei es sich bei dem Verschlussmittel vorzugsweise um ein einrastendes Verschlussmittel handelt, welches insbesondere einen Griff (21) umfasst, und wobei das Kopplungselement (8) und/oder das Unterdrucktherapiegerät (4) eine oder mehrere Ausnehmungen aufweisen, in welche das am Aufnahmebereich (3) vorhandene Verschlussmittel eingreifen kann.

10. Vorrichtung zur Unterdrucktherapie von Wunden nach einem der vorhergehenden Ansprüche, wobei der Aufnahmebereich (3) ein oder mehrere Anschlagelemente (23) aufweist, so dass das Kopplungselement (8) und/oder das Unterdrucktherapiegerät (4) beim Inkontaktbringen mit dem Aufnahmebereich (3) formschlüssig ausgerichtet werden.

11. Vorrichtung zur Unterdrucktherapie von Wunden nach einem der vorhergehenden Ansprüche, wobei der Aufnahmebereich (3) randständig einen teilweise oder vollständig den Aufnahmebereich (3) umlaufenden Steg oder Wulst (22) aufweist, so dass das Kopplungselement (8) und/oder das Unterdrucktherapiegerät (4) beim Inkontaktbringen mit dem Aufnahmebereich (3) formschlüssig ausgerichtet werden.

12. Vorrichtung zur Unterdrucktherapie von Wunden nach einem der vorhergehenden Ansprüche, wobei das Drehgelenk mittels einer Drehsperre in 0°-, 45°-, 90°-, 135°-, 180°, 225°-, 270°, 315°- Stellung lösbar einrasten kann, zur Anbringung der Befestigungsvorrichtung (10) an einer vertikal angeordneten, an einer horizontal angeordneten oder an einer schräg angeordneten Stange oder Platte.

13. Vorrichtung zur Unterdrucktherapie von Wunden nach einem der vorhergehenden Ansprüche, wobei es sich bei dem Unterdrucktherapiegerät (4) um ein am Körper eines Benutzers tragbares Unterdrucktherapiegerät handelt.

14. Verfahren zum Befestigen eines Unterdrucktherapiegerätes, umfassend
- Bereitstellen einer Vorrichtung zur Unterdrucktherapie von Wunden nach einem der vorhergehenden Ansprüche,
- Fixieren der Befestigungsvorrichtung (10) an einem Gegenstand, beispielsweise an einer Platte oder an einer Stange,
- Verdrehen des Aufnahmebereiches (3) gegenüber dem Haltemittel (1), so dass der Aufnahmebereich (3) horizontal ausgerichtet ist,
- Einstecken eines Netzgerätes (6 a) in den Einschub (5) und gegebenenfalls Legen nicht benötigter Kabelabschnitte um eine Kabelaufwicklung (15) des Einschubes (5),
- Einbringen von Stromversorgungskomponenten (6) und/oder Datenkommunikationskomponenten in den Hohlkörper (2), wobei ein Netzkabel, ein Gerätestromversorgungskabel und/oder gegebenenfalls vorhandene Datenkommunikationskabel aus dem Inneren des Hohlkörpers (2) durch Öffnungen (14) im Hohlkörper (2) nach außen geführt werden,
- In Kontakt bringen des Unterdrucktherapiegerätes (4) mit dem Aufnahmebereich (3) der Befestigungsvorrichtung (10),
- Verbinden des Gerätestromversorgungskabels mit dem Unterdrucktherapiegerät (4).

## Claims

1. Device for negative-pressure therapy of wounds, comprising a negative-pressure therapy appliance (4) and a fastening device (10) for the negative-pressure therapy appliance (4), said fastening device comprising
- a holding means (1) for fixing the fastening device (10) on an object, and
- a receiving region (3) for receiving the negative-pressure therapy appliance (4),
**characterized in that**
the fastening device comprises an insert (5) for receiving one or more power supply components (6) and/or data communication components,
and **in that** the fastening device (10) comprises a hollow body (2) into which one or more power supply components (6) and/or data communication components can be fitted, the hollow body (2) comprising one or more openings (14) for the passage of cable sections, and the insert (5) being able to be introduced into the interior of the hollow body (2), and moreover
**in that** the fastening device (10) comprises a pivot joint, such that the receiving region (3) can be rotated relative to the holding means (1).

2. Device for negative-pressure therapy of wounds according to Claim 1, wherein the holding means (1) comprises regions that permit a form-fit connection, a force-fit connection or a cohesively bonded connection of the fastening device to an object, in particular by clamping, latching, hooking, screwing, nailing, clipping, riveting, welding, fusing, adhesion or by magnetic force of attraction.

3. Device for negative-pressure therapy of wounds according to Claim 1 or 2, wherein the holding means (1) comprises a clamping mechanism, such that the fastening device (10) can be fixed with a force fit on an object, in particular on a bar, on a strut, on a panel or on a projecting region.

4. Device for negative-pressure therapy of wounds according to one of the preceding claims, wherein the holding means (1) comprises a holding clip, a stirrup clip, a band clip, a snap-on clip or a C-clamp, in particular a screw clamp.

5. Device for negative-pressure therapy of wounds according to one of the preceding claims, wherein the power supply component (6) is a power pack (6a) and/or a section of a cable and/or an electrical plug connection.

6. Device for negative-pressure therapy of wounds according to one of the preceding claims, wherein the insert (5) comprises a cable winding (15).

7. Device for negative-pressure therapy of wounds according to one of the preceding claims, wherein the receiving region (3) has a shape complementing the negative-pressure therapy appliance (4), such that the negative-pressure therapy appliance (4) is held substantially captive in the receiving region (3).

8. Device for negative-pressure therapy of wounds according to one of the preceding claims, further comprising a coupling element (8) which is releasably connectable or non-releasably connected to the negative-pressure therapy appliance (4), wherein the receiving region (3) can receive the coupling element (8).

9. Device for negative-pressure therapy of wounds according to one of the preceding claims, wherein the receiving region (3) comprises a closure means, such that the negative-pressure therapy appliance (4) can be locked releasably on the fastening device (10), wherein the closure means is preferably a latching closure means, which in particular comprises a grip (21), and wherein the coupling element (8) and/or the negative-pressure therapy appliance (4) have one or more recesses into which the closure means present on the receiving region (3) can engage.

10. Device for negative-pressure therapy of wounds according to one of the preceding claims, wherein the receiving region (3) has one or more abutment elements (23), such that the coupling element (8) and/or the negative-pressure therapy appliance (4) are oriented with a form fit when brought into contact with the receiving region (3).

11. Device for negative-pressure therapy of wounds according to one of the preceding claims, wherein the edge of the receiving region (3) has a web or bead (22) extending partially or completely around the receiving region (3), such that the coupling element (8) and/or the negative-pressure therapy appliance (4) are oriented with a form fit when brought into contact with the receiving region (3).

12. Device for negative-pressure therapy of wounds according to one of the preceding claims, wherein the pivot joint can lock releasably, by means of a rotary lock, at the 0°, 45°, 90°, 135°, 180°, 225°, 270° or 315° position, in order to mount the fastening device (10) on a vertically arranged, a horizontally arranged or an obliquely arranged bar or panel.

13. Device for negative-pressure therapy of wounds according to one of the preceding claims, wherein the negative-pressure therapy appliance (4) is a negative-pressure therapy appliance that can be worn on the body of a user.

14. Method for fastening a negative-pressure therapy appliance, which method comprises
- providing a device for negative-pressure therapy of wounds according to one of the preceding claims,
- fixing the fastening device (10) on an object, for example on a panel or on a bar,
- rotating the receiving region (3) relative to the holding means (1), such that the receiving region (3) is oriented horizontally,
- introducing a power pack (6a) into the insert (5) and, if appropriate, placing unneeded cable sections around a cable winding (15) of the insert (5),
- introducing power supply components (6) and/or data communication components into the hollow body (2), wherein a mains cable, an appliance power supply cable and/or optional data communication cables are guided out of the interior of the hollow body (2) through openings (14) in the hollow body (2),
- bringing the negative-pressure therapy appliance (4) into contact with the receiving region (3) of the fastening device (10),
- connecting the appliance power supply cable to the negative-pressure therapy appliance (4).

## Revendications

1. Dispositif de thérapie de plaies par pression négative, le dispositif comprenant un appareil de thérapie par pression négative (4) et un dispositif de fixation (10) destiné à l'appareil de thérapie par pression négative (4), le dispositif de fixation comprenant
- un moyen de retenue (1) destiné à fixer le dispositif de fixation (10) à un objet, et
- une zone de réception (3) destinée à recevoir l'appareil de thérapie par pression négative (4), **caractérisé en ce que**
le dispositif de fixation comprend un insert (5) destiné à recevoir un ou plusieurs composants d'alimentation en courant (6) et/ou des composants de communication de données,
et le dispositif de fixation (10) comprend un corps creux (2) dans lequel un ou plusieurs composants d'alimentation en courant (6) et/ou composants de communication de données peuvent être placés, le corps creux (2) comprenant une ou plusieurs ouvertures (14) destinées au passage de portions de câble, l'insert (5) pouvant être introduit dans l'espace intérieur du corps creux (2),
et **caractérisé en outre en ce que**
le dispositif de fixation (10) comprend une articulation rotative de manière à pouvoir faire tourner la zone de réception (3) par rapport au moyen de retenue (1).

2. Dispositif de thérapie de plaies par pression négative selon la revendication 1, le moyen de retenue (1) comprenant des zones qui permettent une fixation par liaison par complémentarité de formes, par liaison en force ou par liaison de matière du dispositif de fixation à un objet, notamment par serrage, encliquetage, accrochage, vissage, clouage, agrafage, rivetage, soudage, fusion, collage ou par force d'attraction magnétique.

3. Dispositif de thérapie de plaies par pression négative selon la revendication 1 ou 2, le moyen de retenue (1) comprenant un mécanisme de serrage de sorte que le dispositif de fixation (10) puisse être fixé par une liaison en force à un objet, notamment à une barre, à un montant, à une plaque ou une zone en saillie.

4. Dispositif de thérapie de plaies par pression négative selon l'une des revendications précédentes, le moyen de retenue {1) comprenant une bride de retenue, une bride à étrier, une bride à bande, une borne à ressort ou un serre-joint, notamment un serre-joint à vis.

5. Dispositif de thérapie de plaies par pression négative selon l'une des revendications précédentes, le composant d'alimentation en courant (6) étant un bloc d'alimentation (6a) et/ou une portion d'un câble et/ou un connecteur à enfichage électrique.

6. Dispositif de thérapie de plaies par pression négative selon l'une des revendications précédentes, l'insert (5) comprenant un enrouleur de câble (15).

7. Dispositif de thérapie de plaies par pression négative selon l'une des revendications précédentes, la zone de réception (3) ayant une forme complémentaire de celle de l'appareil de thérapie par pression négative (4) de sorte que l'appareil de thérapie par pression négative (4) est maintenu en grande partie de manière immobile dans la zone de réception (3).

8. Dispositif de thérapie de plaies par pression négative selon l'une des revendications précédentes, comprenant en outre un élément d'accouplement (8) qui peut être relié de manière amovible ou qui est relié de manière permanente à l'appareil de thérapie par pression négative (4), la zone de réception (3) pouvant recevoir l'élément d'accouplement (8).

9. Dispositif de thérapie de plaies par pression négative selon l'une des revendications précédentes, la zone de réception (3) comprenant un moyen de fermeture de sorte que l'appareil de thérapie par pression négative (4) puisse être bloqué de manière libérable sur le dispositif de fixation (10), le moyen de fermeture étant de préférence un moyen de fermeture par encliquetage qui comprend notamment une poignée (21) et l'élément d'accouplement (8) et/ou l'appareil de thérapie par pression négative (4) comportant un ou plusieurs évidements dans lesquels peut s'engager le moyen de fermeture présent sur la zone de réception (3).

10. Dispositif de thérapie de plaies par pression négative selon l'une des revendications précédentes, la zone de réception (3) comportant un ou plusieurs éléments de butée (23) de sorte que l'élément d'accouplement (8) et/ou l'appareil de thérapie par pression négative (4) sont orientés de manière à former une liaison par complémentarité de formes lorsqu'ils sont mis en contact avec la zone de réception (3).

11. Dispositif de thérapie de plaies par pression négative selon l'une des revendications précédentes, la zone de réception (3) comportant en bordure une nervure ou un renflement (22) qui entoure partiellement ou complètement la zone de réception (3) de sorte que l'élément d'accouplement (8) et/ou l'appareil de thérapie par pression négative (4) sont orientés de manière à former une liaison par complémentarité de formes lorsqu'ils sont mis en contact avec la zone de réception (3) .

12. Dispositif de thérapie de plaies par pression négative selon l'une des revendications précédentes, l'articulation rotative pouvant s'encliqueter de manière libérable en position 0°, 45°, 90°, 135°, 180°, 225°, 270°, 315° au moyen d'un système de blocage en rotation pour fixer le dispositif de fixation (10) à une barre ou une plaque disposée verticalement, horizontalement ou de manière inclinée.

13. Dispositif de thérapie de plaies par pression négative selon l'une des revendications précédentes, le dispositif de thérapie par pression négative (4) étant un appareil de thérapie par pression négative qui peut être porté sur le corps d'un utilisateur.

14. Procédé de fixation d'un appareil de thérapie par pression négative, le procédé comprenant les étapes suivantes
- fournir un dispositif de thérapie de plaies par pression négative selon l'une des revendications précédentes,
- fixer le dispositif de fixation (10) à un objet, par exemple à une plaque ou à une barre,
- faire tourner la zone de réception (3) par rapport au moyen de retenue (1) de sorte que la zone de réception (3) soit orientée horizontalement,
- enficher un bloc d'alimentation (6a) dans l'insert (5) et éventuellement placer des portions de câble non nécessaires autour d'un enrouleur de câble (15) de l'insert (5),
- introduire des composants d'alimentation en courant (6) et/ou des composants de communication de données dans le corps creux (2), un câble d'alimentation, un câble d'alimentation d'appareil en courant et/ou éventuellement des câbles de communication de données présents étant guidés depuis l'intérieur du corps creux (2) vers l'extérieur par des ouvertures (14) ménagées dans le corps creux (2),
- mettre l'appareil de thérapie par pression négative (4) en contact avec la zone de réception (3) du dispositif de fixation (10),
- relier le câble d'alimentation d'appareil en courant à l'appareil de thérapie par pression négative (4).
